Europäisches Patentamt

**European Patent Office**

**Office européen des brevets**

(11) Numéro de publication: **0 017 694**
**A1**

## DEMANDE DE BREVET EUROPEEN

(21) Numéro de dépôt: **79430010.3**

(22) Date de dépôt: **13.04.79**

(51) Int. Cl.³: **A 23 L 1/237**, A 61 K 7/50, C 01 B 3/06

(43) Date de publication de la demande: **29.10.80** Bulletin 80/22

(71) Demandeur: **BONETTI Frères Société anonyme:, Zone Industrielle Bois De l'Abbesse, F-68160 Liepvre (FR)**

(72) Inventeur: **Aubert, Maurice, Villa "Lou Mazet" Avenue Henri Vial, F-06 Cros de Cagnes (FR)**

(84) Etats contractants désignés: **BE CH DE GB IT LU NL SE**

(74) Mandataire: **Hautier, Jean-Louis et al, Cabinet Hautier 24 rue Masséna, F-06000 NICE (FR)**

(54) Sels marins enrichis en produits biologiques océaniques; procédé de fabrication.

(57) L'invention a pour objet un procédé de fabrication et des sels marins enrichis en produits biologiques issus dudit procédé.

On utilise de l'eau de mer dans un bac, on ajoute des substances nutritives telles que des nitrates ou phosphates, on ensemence ledit bac avec des cellules de phytoplancton; au bout de dix jours, lorsque le nombre de cellules phytoplanctoniques s'est accru considérablement, on filtre le milieu de culture pour le débarasser des cellules, le filtrat ne contient alors que les sécrétions desdites cellules et est désséché sous vide et à une température moyenne de 40 à 50°C jusqu'à l'otention de sels, lesdits sels sont alors enrichis par les sécrétions desdites cellules et sont prêts à l'emploi.

L'invention s'applique notamment comme sels de bains, comme complément alimentaire, comme élément adjuvant pour la préparation de produits cosmétiques.

- 1 -

<u>Sels marins enrichis en produits biologiques océaniques.</u>

La présente invention a pour objet le procédé de fabrication de sels marins enrichis en produits biologiques océaniques.

Les produits biologiques sont issus du métabolisme planctonique.

Cette invention complète et apporte des perfectionnements
aux techniques décrites dans le brevet déposé le 31.8.1956
et délivré sous le n° 1.164.561 au nom du même auteur.

Le procédé de fabrication de sels marins enrichis en produits biologiques consiste à utiliser de l'eau de mer dans
un bac, puis l'on ajoute des substances nutritives dans ledit bac tels que des nitrates ou des phosphates, puis on
ensemence ledit bac avec les cellules de phytoplancton , au
bout d'une dizaine de jours, lorsque le nombre de cellules
phytoplanctoniques s'est accru considérablement, on filtre
le milieu de culture et l'on récupère le filtrat.

Le filtrat est alors désséché sous vide à température moyenne de 40 à 50°C jusqu'à obtention de sels. Les dits sels
sont alors prêts à l'emploi.

Les cellules de phytoplancton plus particulièrement utilisées peuvent être des diatomées. Les diatomées utilisées peuvent être de préférence, Chaetoceros teres ou Asterionella japonica.

Il est possible, bien entendu,de n'utiliser que Chaetoceros teres ou que Asterionella japonica.

Il est également possible d'utiliser à la fois Chaetoceros teres et Asterionella japonica.

On prend dans un bac de l'eau de mer à laquelle on ajoute des substances nutritives tels que des nitrates ou des phosphates.

Puis, on ensemence ce bac avec des cellules de phytoplancton, plus particulièrement des Diatomées se reproduisant rapidement, par exemple, Chaetoceros teres et Asterionella japonica.

Au bout d'une dizaine de jours, le nombre des cellules phytoplanctoniques s'est accru considérablement, passant par exemple, de 50 cellules par millilitre à 600.000.

On filtre alors le milieu de culture, c'est-à-dire l'eau de mer où ont vécu les cellules phytoplanctoniques et qui s'est enrichi de toutes les substances secrétées par ces micro-organismes planctoniques : matières organiques diverses, acides gras, protéines, glucides...

Le milieu de culture est filtré sur un premier filtre en nylon dont les mailles ont 100 $\mu$ de diamètre, puis sur un second filtre dont le diamètre des mailles est nettement plus petit, entre 10 et 0,5 $\mu$

On comprend donc qu'ainsi, on utilise bien l'eau de mer et les sécrétions des cellules de diatomées pour enrichir les sels et qu'en aucun cas, on utilise directement les organismes vivants.

Le filtrat est alors désséché sous vide et à température moyenne de 40 à 50°, jusqu'à obtention de cristaux de sels.

L'analyse de ce sel montre qu'il contient les mêmes substances organiques que celles existant dans l'eau de mer d'où il est issu et que les organismes planctoniques ont apporté et par ailleurs que les propriétés physiologiques afférentes à ces substances sont conservées.

Les sels marins enrichis ainsi obtenus sont utilisables :

- soit comme sels de bains qui, une fois redissous dans l'eau du bain lui apportent des qualités agréables : odeur marine, consistance émolliente, ainsi que des propriétés bénéfiques pour les tissus cutanés;

- soit comme complément alimentaire.

En effet, l'apport de substances organiques provenant de la vie planctonique, va donner à ce sel marin un supplément d'activité, favorisant les métabolismes somatiques par la présence de substances synthétisées par les cellules planctoniques telles que des vitamines.

- soit comme élément adjuvant pour la préparation de produits cosmétiques, ce sel riche en oligo-éléments marins et en substances organiques produites naturellement, étant doué d'activité trophique vis-à-vis des cellules épidermiques.

Revendications de brevet

1. Procédé de fabrication de sels marins enrichis en produits et sécrétions biologiques, caractérisé par le fait que l'on utilise de l'eau de mer dans un bac, que l'on ajoute des substances nutritives telles que des nitrates ou phosphates, que l'on ensemence ledit bac avec des cellules de phytoplancton, qu'au bout de dix jours, lorsque le nombre de cellules phytoplanctoniques s'est accru considérablement, on filtre le milieu de culture pour le débarasser des cellules, le filtrat ne contient alors que les sécrétions desdites cellules et est désséché sous vide et à température moyenne de 40 à 50°C jusqu'à l'obtention de sels, lesdits sels sont alors enrichis par les sécrétions desdites cellules et sont prêts à l'emploi.

2. Procédé de fabrication de sels marins enrichis en produits et sécrétions biologiques selon la revendication 1, caractérisé par le fait que les sécrétions des cellules de phytoplancton utilisées sont les sécrétions des cellules des diatomées.

3. Procédé de fabrication de sels marins enrichis en produits et sécrétions biologiques, selon l'une quelconque des revendications 1 ou 2, caractérisé par le fait que les sécrétions des cellules des diatomées utilisées, sont des cellules de Chaetoceros teres.

4. Procédé de fabrication de sels marins enrichis en produits et sécrétions biologiques selon l'une quelconque des revendications 1 ou 2, caractérisé par le fait que les sécrétions de diatomées utilisées sont des sécrétions de Asterionella japonica.

- 2 -

5. Procédé de fabrication de sels marins enrichis en produits et sécrétions biologiques selon l'une quelconque des revendications 1 ou 2, caractérisé par le fait que les sécrétions de diatomées utilisées sont à la fois Chaetoceros teres et Asterionella japonica.

6. Sels marins enrichis en produits et sécrétions biologiques obtenus selon l'une quelconque des revendications 1,2,3,4 ou 5.

7. Sels marins selon la revendication 6, enrichis en produits et sécrétions biologiques, caractérisé par le fait que les sels marins sont enrichis uniquement par les sécrétions des cellules de diatomées rejetées dans le milieu de culture.

8. Procédé selon l'une quelconque des revendications 1,2, 3,4 ou 5, caractérisé par le fait que le milieu de culture est filtré sur un premier filtre en nylon dont les mailles ont 100 $\mu$ de diamètre, puis sur un second filtre dont le diamètre des mailles est nettement plus petit, entre 10 et 0,5 $\mu$ de diamètre, de manière à être enrichi par les sécrétions desdites cellules de phytoplancton.

# 0017694
Numero de la demande

**RAPPORT DE RECHERCHE EUROPEENNE**

Office européen
des brevets

EP 79 43 0010

## DOCUMENTS CONSIDERES COMME PERTINENTS

| Catégorie | Citation du document avec indication, en cas de besoin, des parties pertinentes | Revendication concernée |
|---|---|---|
| | <u>FR - A - 2 165 154</u> (AUBERT) <br> * Revendications 1-3; page 1, lignes 16-36 * <br><br> -- | 1-8 |
| A | <u>FR - A - 2 388 507</u> (J.P. RICHARD) | |
| AD | <u>FR - A - 1 164 561</u> (AUBERT) <br><br> ---- | |

**CLASSEMENT DE LA DEMANDE (Int. Cl. ³)**

A 23 L 1/237
A 61 K 7/50
C 01 B 3/06

**DOMAINES TECHNIQUES RECHERCHES (Int. Cl. ³)**

A 23 L 1/237
A 23 L 1/337
A 61 K 7/50
C 01 B 3/06

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le present rapport de recherche a été etabli pour toutes les revendications

| Lieu de la recherche | Date d'achevement de la recherche | Examinateur |
|---|---|---|
| La Haye | 12-12-1979 | VAN MOER |

OEB Form 1503.1  06.78